# EUROPEAN PATENT SPECIFICATION

(11) **EP 4 066 730 B1**
(45) Date of publication and mention of the grant of the patent: **14.02.2024**
(21) Application number: 20910189.8
(22) Date of filing: 15.12.2020
(51) Int. Cl.: A61B 5/00, A61B 5/11, A61B 5/024

(54) **DATA ACQUISITION METHOD, TERMINAL DEVICE, AND STORAGE MEDIUM**
DATENERFASSUNGSVERFAHREN, ENDGERÄT UND SPEICHERMEDIUM
PROCÉDÉ D'ACQUISITION DE DONNÉES, DISPOSITIF DE TERMINAL ET SUPPORT DE STOCKAGE

(30) Priority: 30.12.2019 CN 201911392230
(43) Date of publication of application: 05.10.2022
(73) Proprietor: Huawei Technologies Co., Ltd., Shenzhen, Guangdong 518129, (CN)
(72) Inventor: HUANG, Xi, Shenzhen, Guangdong 518129 (CN); WU, Huangwei, Shenzhen, Guangdong 518129 (CN)
(74) Representative: Gill Jennings & Every LLP
(86) International application number: PCT/CN2020/136536
(87) International publication number: WO 2021/135932

(56) References cited:
- WO-A1-2018/113119
- WO-A1-2018/113119
- CN-A- 107 647 850
- CN-A- 107 951 485
- CN-A- 108 289 621
- CN-A- 108 697 329
- CN-A- 109 758 140
- US-A1- 2017 172 463
- US-A1- 2018 353 107
- US-A1- 2019 110 755

## Description

### TECHNICAL FIELD

This application relates to the field of electronic technologies, and in particular, to a data collection method, a terminal device, and a storage medium.

### BACKGROUND

With rapid development of electronic technologies, a wearable device can implement more functions. For example, the wearable device can collect various human physiological data, such as an electrocardio signal and a blood oxygen signal of a human body. When a conventional wearable device collects human physiological data, it is required that a collected part of a human body be in continuous contact with an area in which a sensor is located for preset duration, to obtain available collected data. If the collected part of the human body is in poor contact with the area in which the sensor is located in the preset duration, invalid data exists in the collected data. When duration of the invalid data in the collected data exceeds a preset threshold, the conventional wearable device discards the collected data, and therefore the human physiological data of a user needs to be re-collected based on the preset duration until the re-collected data is available. It can be learned that the data collection process of the conventional wearable device has a high user operation requirement, and a collection success rate of the available data is low. Consequently, a total quantity of data collection times is large, and total data collection duration is long.

Document US 2019/110755 A1 discloses a method for detecting heart rate variability in sleep states. Document WO 2018/113119 A1 also relates to the field of sleep monitoring and discloses a data synchronisation method between a first terminal device configured collect physiological data or motion data of a user to perform sleep stage analysis and a second terminal device.

### SUMMARY

Implementations of this application provide a data collection method, a terminal device, and a storage medium, which can be used to reduce a user operation requirement in a data collection process, improve a collection success rate of target collected data for data analysis, reduce a total quantity of data collection times, and shorten total data collection duration.

The invention has been defined in the independent claims. Further specific technical features have been defined in the dependent claims.

Compared with the conventional technology, implementations of this application have the following beneficial effects.

According to the data collection method provided in implementations of this application, the original collected data is not discarded provided that the original collected data includes a valid data segment. In other words, a status in which the collected part of the human body is in contact with the sensor in the data collection process is not strictly limited in implementations of this application, provided that the original collected data includes a valid data segment. This reduces a user operation requirement in the data collection process. In implementations of this application, the at least one piece of original collected data of the target user collected by the sensor in the first preset time period is obtained; the at least one target valid data segment whose total duration meets the preset duration requirement is selected from the at least one piece of original collected data; and the target collected data of the target user is obtained based on the target valid data segment. In this way, available target collected data can be obtained based on valid data segments in a plurality of pieces of original collected data, so that a collection success rate of the available target collected data is improved and a total quantity of data collection times is reduced. Compared with the conventional technology, in this application, valid data in original collected data obtained through each collection may be used as a part of the available target collected data, so that duration of each data collection is not strictly limited and total duration of data collection is shortened.

### BRIEF DESCRIPTION OF DRAWINGS

FIG. 1 is a schematic diagram of a hardware structure of a terminal device to which a data collection method is applied according to an implementation of this application;
FIG. 2 is a block diagram of a structure of a data collection system according to an implementation of this application;
FIG. 3 is a schematic flowchart of a data collection method according to an implementation of this application;
FIG. 4 is a schematic diagram of a data structure of at least one piece of original collected data in a first preset time period according to an implementation of this application;
FIG. 5 is a schematic diagram of a data structure of a target collected data obtained by splicing at least one target valid data segment according to an implementation of this application;
FIG. 6 is a schematic flowchart of a data collection method according to another implementation of this application;
FIG. 7 is a schematic waveform diagram of a piece of original collected data collected by a sensor according to an implementation of this application;
FIG. 8 is a schematic flowchart of a data collection method according to still another implementation of this application;
FIG. 9 is a schematic flowchart of a data collection method according to still another implementation of this application;
FIG. 10 is a schematic waveform diagram of preset human physiological data and motion data that is of a collected part of a human body and that is collected together with the human physiological data according to an implementation of this application;
FIG. 11 is a schematic flowchart of a data collection method according to another implementation of this application;
FIG. 12 is a schematic flowchart of a data collection method according to another implementation of this application;
FIG. 13 is a block diagram of a structure of a terminal device according to an implementation of this application; and
FIG. 14 is a schematic diagram of a structure of a terminal device according to another implementation of this application.

### DESCRIPTION OF IMPLEMENTATIONS

To make the objectives, technical solutions, and advantages of this invention clearer, the following further describes various implementations in detail with reference to the accompanying drawings. The implementations described below are not all claimed, they are included to help understanding the context of the invention. While the description refers to various implementations, the embodiments of the invention are those that comprise at least all the features of an independent claim. Any implementation that does not fall within the scope of the claims does not form part of the invention, but rather included as an illustrative example that is useful for understanding the invention.

It should be understood that, when used in the specification and claims of this application, the term "include" indicates presence of described features, entireties, steps, operations, elements, and/or components, but does not exclude presence or addition of one or more other features, entireties, steps, operations, elements, components, and/or collections thereof.

It should be further understood that the term "and/or" used in the specification and claims of this application indicates any combination and all possible combinations of one or more items listed in association, and includes the combinations.

As used in the specification and claims of this application, the term "if" may be interpreted as "when", "once", "in response to determining", or "in response to detecting" depending on the context. Similarly, the phrase "if it is determined" or "if (the described condition or event) is detected" may be interpreted as a meaning of "when it is determined that" or "in response to determining" or "when (the described condition or event) is detected" or "in response to detecting (the described condition or event)" depending on the context.

In addition, in the descriptions of the specification and claims of this application, the terms "first", "second", "third", and the like are merely intended for differentiated description, but shall not be understood as an indication or an implication of relative importance.

Reference to "an implementation", "some implementations" or the like in the specification of this application means that one or more implementations of this application include a specific feature, structure, or characteristic described with reference to the implementation. Therefore, statements such as "in an implementation", "in some implementations", "in some other implementations", and "in other implementations" that appear at different places in this specification do not necessarily mean referring to a same implementation, but mean "one or more but not all of the implementations", unless otherwise specifically emphasized. The terms "include", "comprise", "have", and their variants all mean "include but are not limited to", unless otherwise specifically emphasized.

The data collection method provided in implementations of this application may be applied to a terminal device. The terminal device may be a wearable device or may be a mobile terminal such as a mobile phone, a tablet computer, a vehicle-mounted device, an augmented reality (augmented reality, AR)/virtual reality (virtual reality, VR) device, a notebook computer, an ultra-mobile personal computer (ultra-mobile personal computer, UMPC), a netbook, or a personal digital assistant (personal digital assistant, PDA). A specific type of the terminal device is not limited in implementations of this application.

For example, the terminal device may be specifically a cellular phone, a cordless phone, a session initiation protocol (Session Initiation Protocol, SIP) phone, a personal digital assistant (Personal Digital Assistant, PDA) device, a handheld device having a wireless communication function, a computing device or another processing device connected to a wireless modem, a handheld communications device, a handheld computing device, and/or another device for network communication in a wireless system and a next-generation communications system, for example, a mobile terminal in a 5G network or a mobile terminal in a future evolved public land mobile network (Public Land Mobile Network, PLMN).

As an example rather than a limitation, when the terminal device is a wearable device, the wearable device may alternatively be a generic term for wearable devices such as glasses, gloves, watches, clothes, and shoes that are developed based on intelligent design of daily wearing by using wearable technologies. The wearable device is a portable device that can be directly worn by a user or integrated into clothes or an accessory of the user. The wearable device is more than a hardware device, and is used to implement a powerful function through software support, data exchange, and cloud interaction. In a broad sense, the wearable intelligent devices include full-featured and large-sized devices that can implement complete or partial functions without depending on smartphones, such as smart watches and smart glasses, and devices that focus on only one type of application function and need to work with other devices such as smartphones, such as various smart bands and smart jewelry for collection of human physiological data, collection of human motion data, and monitoring of vital signs.

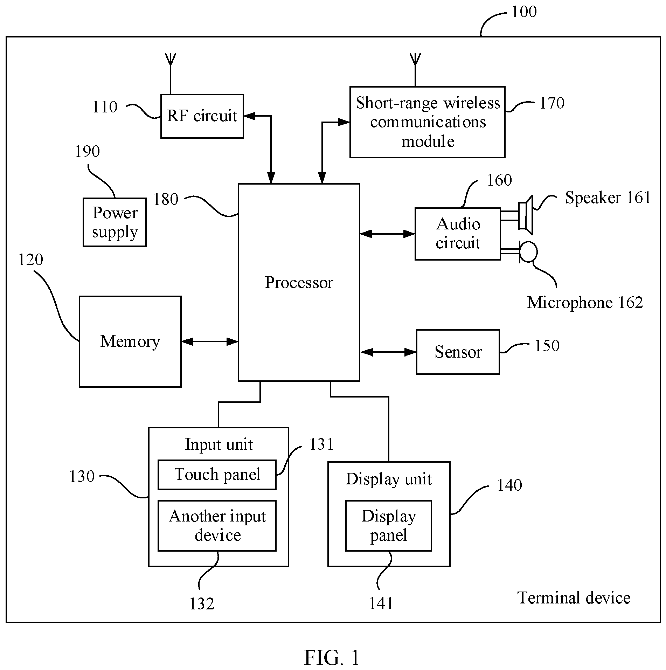

FIG. 1 is a schematic diagram of a hardware structure of a terminal device to which a data collection method is applied according to an implementation of this application. As shown in FIG. 1, a terminal device 100 may be a wearable device, or may be a mobile terminal such as a mobile phone or a tablet computer. The mobile phone 100 may specifically include components such as a radio frequency (Radio Frequency, RF) circuit 110, a memory 120, an input unit 130, a display unit 140, a sensor 150, an audio circuit 160, a short-range wireless communications module 170, a processor 180, and a power supply 190. It can be understood by persons skilled in the art that a structure of the terminal device 100 shown in FIG. 1 does not constitute any limitation on the terminal device, and the terminal device may include more or fewer components than those shown in the figure, or have some components combined, or have different component arrangements.

The following specifically describes each constituent component of the terminal device 100 in detail with reference to FIG. 1.

The RF circuit 110 may be configured to: receive and send a signal during information receiving and sending or during a call; particularly, send downlink information to the processor 180 for processing after receiving the downlink information of a base station; and in addition, send related uplink data to the base station. Usually, the RF circuit includes but is not limited to an antenna, at least one amplifier, a transceiver, a coupler, a low noise amplifier (Low Noise Amplifier, LNA), a duplexer, and the like. In addition, the RF circuit 110 may further communicate with a network and another device through wireless communication. The foregoing wireless communication may use any communications standard or protocol, which includes but is not limited to a Global System for Mobile Communications (Global System for Mobile communications, GSM), a general packet radio service (General Packet Radio Service, GPRS), Code Division Multiple Access (Code Division Multiple Access, CDMA), Wideband Code Division Multiple Access (Wideband Code Division Multiple Access, WCDMA), Long Term Evolution (Long Term Evolution, LTE), an email, a short message service (Short Message Service, SMS), or the like.

The memory 120 may be configured to store a software program and a module, and the processor 180 executes various functional applications of the terminal device 100 and data processing by running the software program and the module that are stored in the memory 120. The memory 120 may mainly include a program storage area and a data storage area. The program storage area may store an operating system, an application required by at least one function (such as a human physiological data analysis function and a human motion data analysis function), and the like. The data storage area may store data (such as human motion data and motion data of a collected part of a human body) created based on use of the terminal device 100, and the like. For example, when the terminal device 100 is a wearable device, the wearable device may store, in a data storage area of the wearable device, original collected data collected by a sensor. When the terminal device 100 is a mobile terminal such as a mobile phone or a tablet computer, the mobile terminal may receive original collected data sent by a wearable device, and store the original collected data in a data storage area of the mobile terminal. In addition, the memory 120 may include a highspeed random access memory, or may include a nonvolatile memory such as at least one magnetic disk storage device, a flash memory device, or another nonvolatile solid-state storage device.

The input unit 130 may be configured to: receive entered digital or character information, and generate a button signal input related to user setting and function control of the terminal device 100. Specifically, the input unit 130 may include a touch panel 131 and another input device 132. The touch panel 131, also referred to as a touchscreen, may collect a touch operation of a user on or near the touch panel 131 (such as an operation of the user on or near the touch panel 131 by using any suitable object or attachment such as a finger or a stylus), and drive a corresponding connection apparatus according to a preset program.

The display unit 140 may be configured to display information entered by the user or information provided for the user, and various menus of the terminal device 100, for example, output a received electrocardio signal. The display unit 140 may include a display panel 141. Optionally, the display panel 141 may be configured in a form of a liquid crystal display (Liquid Crystal Display, LCD), an organic light-emitting diode (Organic Light-Emitting Diode, OLED), or the like. Further, the touch panel 131 may cover the display panel 141. When detecting a touch operation on or near the touch panel 131, the touch panel 131 transfers the touch operation to the processor 180 to determine a type of a touch event, and then the processor 180 provides corresponding visual output on the display panel 141 based on the type of the touch event. Although the touch panel 131 and the display panel 141 in FIG. 1 are used as two independent components to implement input and output functions of the terminal device 100, in some implementations, the touch panel 131 and the display panel 141 may be integrated to implement the input and output functions of the terminal device 100.

The terminal device 100 may further include at least one sensor 150. For example, when the terminal device 100 is a mobile terminal such as a mobile phone or a tablet computer, the sensor 150 may include, for example, an optical sensor, a motion sensor, and another sensor. When the mobile terminal 100 is a wearable device, the sensor 150 may include, for example, a motion sensor and an electrocardiography sensor. Specifically, the optical sensor may include an ambient optical sensor and a proximity sensor. The ambient optical sensor may adjust luminance of the display panel 141 according to brightness of ambient light, and the proximity sensor may turn off the display panel 141 and/or backlight when the mobile terminal moves to an ear. As one type of the motion sensor, an accelerometer sensor may detect values of acceleration in various directions (usually on three axes). The accelerometer sensor may detect a value and a direction of gravity when the accelerometer sensor is in a static state, and may be used on an application for recognizing a terminal device 100 posture (such as switching between a landscape screen and a portrait screen, a related game, and magnetometer posture calibration), a function related to vibration recognition (such as a pedometer and a knock), and the like. Other sensors such as a gyroscope, a barometer, a hygrometer, a thermometer, and an infrared sensor may be further configured in the terminal device 100. Details are not described herein again.

The audio circuit 160, a speaker 161, and a microphone 162 can provide an audio interface between the user and the terminal device 100. The audio circuit 160 may convert received audio data into an electrical signal and then transmit the electrical signal to the speaker 161, and the speaker 161 converts the electrical signal into a sound signal for output. In addition, the microphone 162 converts a collected sound signal into an electrical signal. The audio circuit 160 receives the electrical signal, converts the electrical signal into audio data, and then outputs the audio data to the processor 180 for processing. Then the processor 180 sends the audio data to, for example, another terminal device 100 through the RF circuit 110, or outputs the audio data to the memory 120 for further processing.

The terminal device 100 may perform wireless communication with another device by using the short-range wireless communications module 170. For example, the short-range wireless communications module 170 may be integrated with at least one of a near field communications module, a Bluetooth communications module, a wireless fidelity (Wireless Fidelity, Wi-Fi) module, and the like. For example, when the terminal device 100 is a wearable device, the wearable device may establish a wireless communication connection to a mobile terminal such as a mobile phone or a tablet computer by using a short-range wireless communications module, and send, to the mobile terminal, preset human physiological data and/or motion data of a collected part of a human body that are/is collected by a sensor. When the terminal device 100 is a mobile terminal such as a mobile phone or a tablet computer, the mobile terminal may establish a wireless communication connection to the wearable device by using a short-range wireless communications module, and receive preset human physiological data and/or motion data of a collected part of a human body that are/is sent by a wearable device.

It may be understood that although not shown in FIG. 1, the terminal device 100 may further include a wired communications interface, for example, a universal serial bus (Universal Serial Bus, USB) interface. The terminal device 100 may establish a wired communication connection to another terminal device 100 through the wired communications interface, and perform wired communication. For example, when the terminal device 100 is a wearable device, the wearable device may establish a wired communication connection to a mobile terminal such as a mobile phone or a tablet computer through a USB interface, and send, to the mobile terminal through the USB interface, preset human physiological data and/or motion data of a collected part of a human body that are/is collected by a sensor. When the terminal device 100 is a mobile terminal such as a mobile phone or a tablet computer, the mobile terminal may receive, through a USB interface, preset human physiological data and/or motion data of a collected part of a human body that are/is sent by a wearable device.

The processor 180 is a control center of the terminal device 100, and connects to various parts of the terminal device 100 through various interfaces and lines. The processor 180 performs various functions of the terminal device 100 and data processing by running or executing a software program and/or a module stored in the memory 120 and by invoking data stored in the memory 120, to perform overall monitoring on the terminal device 100. Optionally, the processor 180 may include one or more processing units. Preferably, the processor 180 may integrate an application processor and a modem processor. The application processor mainly processes an operating system, a user interface, an application, and the like. The modem processor mainly processes wireless communication. It may be understood that the foregoing modem processor may alternatively not be integrated into the processor 180.

The terminal device 100 further includes the power supply 190 (for example, a battery) that supplies power to each component. Preferably, the power supply may be logically connected to the processor 180 by using a power management system, to implement functions such as charging, discharging, and power consumption management by using the power management system.

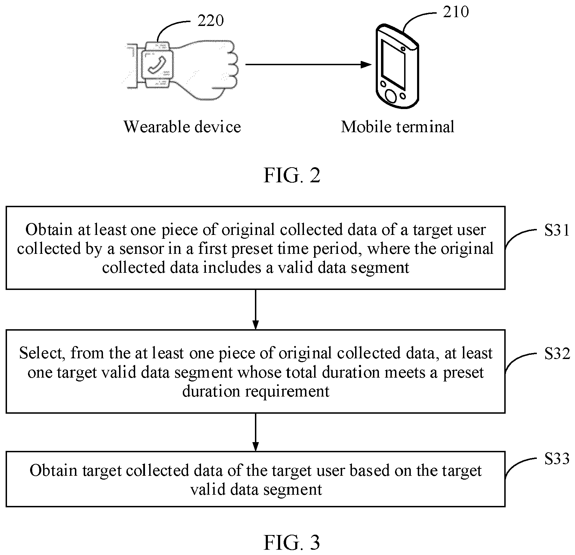

FIG. 2 is a block diagram of a structure of a data collection system according to an implementation of this application. As shown in FIG. 2, the data collection system includes a mobile terminal 210 and a wearable device 220. The wearable device 220 may establish a wireless communication connection to the mobile terminal 210 in a short-range wireless communication manner, or the wearable device 220 may establish a wired communication connection to the mobile terminal 210 in a wired communication manner. A specific communication manner between the wearable device 220 and the mobile terminal 210 is not limited in implementations of this application.

Specifically, after a user wears the wearable device 220 and enables a to-be-detected data collection function of the wearable device 220, the wearable device 220 may collect, by using a built-in sensor configured to collect to-be-detected data, to-be-detected data of the user who wears the wearable device 220. The to-be-detected data may be set based on an actual requirement. For example, the to-be-detected data may be an electrocardio signal of a human body, an acceleration of the human body, or the like. It should be noted that to-be-detected data that is collected by the sensor and that is not processed is original collected data collected by the sensor.

The wearable device 220 may upload the original collected data collected by the sensor to the mobile terminal 210. For example, the wearable device 220 may establish the communication connection to the mobile terminal 210 in the short-range wireless communication manner or the wired communication manner, and upload the original collected data collected by the sensor to the mobile terminal 210. The mobile terminal 210 may store the original collected data from the sensor of the wearable device 220.

The following implementations may be implemented in the terminal device 100 having the foregoing hardware structure/software structure. In the following implementations, the terminal device 100 is used as an example to describe data collection methods provided in implementations of this application.

FIG. 3 is a schematic flowchart of a data collection method according to an implementation of this application. In this implementation, a procedure is executed by a terminal device. As an example rather than a limitation, the terminal device may be the mobile terminal 210 shown in FIG. 2, or may be the wearable device 220.

As shown in FIG. 3, the data collection method provided in this implementation includes S31 to S33. Details are as follows:

S31: Obtain at least one piece of original collected data of a target user collected by a sensor in a first preset time period, where the original collected data includes a valid data segment.

In this implementation, the sensor may be a sensor disposed in a wearable device, and the original collected data is to-be-detected data that is directly collected by the sensor and that is not processed. A specific type of the to-be-detected data may be set based on an actual requirement, and is not limited herein. For example, when a preset physiological feature of a human body needs to be detected, the to-be-detected data may include human physiological data that can represent the preset physiological feature. For example, when an activity feature of a human heart needs to be detected, the to-be-detected data may include an electrocardio signal that can represent the activity feature of the human heart. Alternatively, when a motion status of the human body needs to be detected, the to-be-detected data may further include motion data that can represent the motion status of the human body, for example, an acceleration of the human body.

In specific application, different sensors corresponding to to-be-detected data may be configured in the wearable device based on different types of to-be-detected data. For example, when the to-be-detected data includes an electrocardio signal of the human body, an electrocardiography sensor configured to collect an electrocardio signal of a human body may be configured in the wearable device. The electrocardiography sensor may include but is not limited to a photoplethysmography (Photoplethysmography, PPG) sensor, an electrocardiogram (Electrocardiogram, ECG) sensor, or the like. Specifically, as an example rather than a limitation, when the wearable device is an electronic device that can be in direct contact with skin of the user, such as a band or a watch, and the electrocardiography sensor is the PPG sensor, the PPG sensor may be disposed in an area close to the back of the watch body of the band or the watch. In this way, when the user wears the band or the watch to enable an area on the back of the watch body of the band or the watch in contact with the skin of the user, the PPG sensor in the band or the watch may collect an electrocardio signal of the user based on a blood volume change of a wearing part of the user. Further, when the electrocardio signal of the human body is collected, if the to-be-detected data further includes motion data of the collected part of the human body, a motion sensor configured to collect human motion data may be further configured in the wearable device. The motion sensor may include but is not limited to an acceleration sensor and the like. The collected part of the human body is the part on which the human body wears the wearable device.

In this implementation, when the user wears the wearable device and enables a to-be-detected data collection function of the wearable device, the wearable device may control a corresponding sensor to collect to-be-detected data of the user, the to-be-detected data collected by the sensor is original collected data. For example, when the to-be-detected data includes an electrocardio signal of a human body, the user may enable an electrocardio signal collection function of the wearable device. When detecting that the user enables the electrocardio signal collection function, the wearable device controls the electrocardiography sensor to collect an electrocardio signal of the user. The electrocardio signal of the user collected by the sensor is original collected data. When the to-be-detected data further includes motion data of a collected part of the human body, the user may enable a motion data collection function of the wearable device. When detecting that the user enables the motion data collection function, the wearable device controls the motion sensor to collect motion data of a collected part of the user. It may be understood that, in a data collection process, a contact status between the collected part of the human body and the wearable device may change, and a position status, the contact status, or the like between the collected part of the human body and the sensor changes. Therefore, the to-be-detected data collected by the sensor may include an invalid data segment caused by poor contact. In other words, each piece of original collected data collected by the sensor may further include an invalid data segment in addition to a valid data segment.

The valid data segment and/or the invalid data segment in the original collected data is identified based on data quality of the original collected data. The data quality of the original collected data is specifically described through a preset data feature of the to-be-detected data corresponding to the original collected data, and the preset data feature may be determined based on a type of the to-be-detected data. For example, when the to-be-detected data corresponding to the original collected data includes preset human physiological data such as an electrocardio signal of a human body, a preset data feature of the human physiological data may be a signal-to-noise ratio. In other words, data quality of the human physiological data may be described using the signal-to-noise ratio of the human physiological data. In this case, a data segment whose signal-to-noise ratio meets a preset signal-to-noise ratio requirement in the human physiological data may be identified as a valid data segment, and a data segment whose signal-to-noise ratio does not meet the preset signal-to-noise ratio requirement may be identified as an invalid data segment. In a possible implementation of this implementation, in a to-be-detected data collection process, the wearable device may detect, in real time, data quality of each piece of to-be-detected data (that is, original collected data) collected by the sensor. In another possible implementation of this implementation, the wearable device or the mobile terminal may detect data quality of each piece of original collected data collected by the sensor after to-be-detected data is collected.

It should be noted that, when performing subsequent data analysis, the terminal device needs to perform the data analysis based only on the valid data segment in the original collected data. Therefore, optionally, when identifying that specific original collected data includes only an invalid data segment but does not include a valid data segment, the terminal device may discard the original collected data. In other words, the terminal device may store only original collected data that includes a valid data segment, for subsequent data analysis.

In a possible implementation of this implementation, when the terminal device is the wearable device, that is, when a procedure is executed by the wearable device, the wearable device may store the original collected data from the sensor in a local memory of the wearable device. Based on this, when determining, based on the original collected data, target collected data that is finally used for data analysis, the wearable device may obtain the original collected data from the local memory of the wearable device. In another possible implementation of this implementation, when the terminal device is the mobile terminal such as a mobile phone or a tablet computer, that is, when a procedure is executed by the mobile terminal such as the mobile phone or the tablet computer, the wearable device may upload the original collected data from the sensor to the mobile terminal, and the mobile terminal may store, in a local memory of the mobile terminal, the original collected data sent by the wearable device. Based on this, when determining, based on the original collected data, target collected data that is finally used for data analysis, the mobile terminal may obtain the original collected data from the local memory of the wearable device.

Further, to distinguish between original collected data of different users, the wearable device may further obtain identity information of a collected user when the wearable device collects to-be-detected data of the user by using a sensor. For example, in a possible implementation of this implementation, each time the wearable device collects to-be-collected data of a user, the wearable device may obtain identity information of the to-be-collected user by inquiring the user. For example, the wearable device may output prompt information each time before collecting to-be-detected data, to prompt the user to enter or confirm identity information of the user. In another possible implementation of this implementation, because biometric features of different users are different, the wearable device may further obtain biometric feature information of a collected user each time to-be-detected data is collected, to determine identity information of the collected user by identifying the biometric feature information of the collected user. The biometric feature information may include but is not limited to fingerprint information, iris information, an electrocardio signal, and the like.

In the context of the claimed invention, the to-be-detected data includes the preset human physiological data and the human motion data. Because a physiological status or a motion status of a human body may change in real time, human physiological data or human motion data collected by the sensor at different time points is usually different. Therefore, in an implementation of this application, when collecting the to-be-detected data of the user, the wearable device further records a collection time point of each piece of original collected data corresponding to the to-be-detected data. For example, when the to-be-detected data is an electrocardio signal of a human body, the wearable device further records a collection time point of each electrocardio signal when the wearable device collects the electrocardio signal of the human body. In specific application, a collection start moment of the original collected data may be determined as the collection time point of the original collected data, or a collection end moment of the original collected data may be determined as the collection time point of the original collected data, or any moment between the collection start moment and the collection end moment of the original collected data may be determined as the collection time point of the original collected data. This is specifically determined based on an actual requirement. For example, if a collection start moment of specific original collected data is 13:17:21 on December 11, 2019, a collection end moment is 13:17:49 on December 11, 2019, and duration is 28 seconds, the collection start moment 13:17:21 on December 11, 2019 may be determined as a collection time point of the original collected data, or the collection end moment 13:17:49 on December 11, 2019 may be determined as a collection time of the original collected data, or the like.

In a possible implementation of this implementation, when a procedure is executed by the wearable device, after the wearable device obtains a collection time point of each piece of original collected data and identity information of the user corresponding to each piece of original collected data, the wearable device may store an association between each piece of original collected data, the collection time point of each piece of original collected data, and the corresponding identity information of the user in the local memory of the wearable device. In another possible implementation of this implementation, when a procedure is executed by the mobile terminal such as the phone and the tablet computer, after the wearable device obtains a collection time point of each piece of original collected data and identity information of the user corresponding to each piece of original collected data, the wearable device may upload each piece of original collected data, the collection time point of each piece of original collected data, and the corresponding identity information of the user, and the mobile terminal stores an association between each piece of original collected data, the collection time point of each piece of original collected data, and the corresponding identity information of the user in the local memory of the mobile terminal.

In this implementation, a purpose of storing the original collected data by the terminal device is to subsequently determine, based on the original collected data, the target collected data that is finally used for data analysis. It should be noted that the target collected data used for data analysis includes only the valid data segment, and during data analysis, duration of the target collected data usually needs to meet a preset duration requirement. For example, the duration of the target collected data needs to be greater than or equal to a first preset duration threshold. However, duration of a valid data segment in each piece of original collected data usually does not meet the preset duration requirement. Therefore, when the to-be-detected data of the target user needs to be analyzed, the terminal device may obtain, from the memory of the terminal device, at least one piece of original collected data corresponding to the to-be-detected data of the target user, and determine the target collected data based on the at least one piece of original collected data. The target user may be any user whose to-be-detected data needs to be analyzed.

It may be understood that, as described above, to-be-detected data of a same user collected by the sensor at different time points may be different. For example, an electrocardio signal of the user collected by the sensor on a current day may be different from an electrocardio signal of the user collected on a previous day. Therefore, for a purpose of ensuring accuracy of the to-be-detected data analysis, a first preset time period for limiting an effective collection time interval of the original collected data may be set. Duration of the first preset time period may be set based on an actual requirement. For example, the duration of the first preset time period may be 5 minutes. When determining the target collected data of the target user based on the original collected data of the target user, the terminal device may obtain, from the memory of the terminal device, the at least one piece of original collected data of the target user collected by the sensor in the first preset time period. As an example rather than a limitation, the terminal device may obtain, according to a chronological order of collection time points from the memory of the terminal device, the at least one piece of original collected data of the target user collected by the sensor. In addition, a time interval between first original collected data with an earliest collection time point in the at least one piece of original collected data and second original collected data with a latest collection time point in the at least one piece of original collected data is enabled to be less than or equal to target duration corresponding to the first preset time period.

In a possible implementation of this implementation, a time interval between a collection start moment of the first original collected data and a collection start moment of the second original collected data may be determined as the time interval between the first original collected data and the second original collected data. In another possible implementation of this implementation, a time interval between a collection end moment of the first original collected data and a collection end moment of the second original collected data may alternatively be determined as the time interval between the first original collected data and the second original collected data. Alternatively, in another possible implementation of this implementation, a time interval between a collection start moment of the first original collected data and a collection end moment of the second original collected data may be determined as the time interval between the first original collected data and the second original collected data. Alternatively, in another possible implementation of this implementation, a time interval between a collection end moment of the first original collected data and a collection start moment of the second original collected data may be determined as the time interval between the first original collected data and the second original collected data. This may be specifically determined based on an actual requirement, and is not limited herein.

S32: Select, from the at least one piece of original collected data, at least one target valid data segment whose total duration meets the preset duration requirement.

In a possible implementation of this implementation, that total duration meets the preset duration requirement may be specifically that the duration is greater than or equal to the first preset duration threshold. The first preset duration threshold is used to describe a minimum data amount needed for data analysis. The first preset duration threshold may be set based on an actual requirement. For example, the first preset duration threshold may be 30 seconds. In other words, the minimum data amount needed for data analysis is 30 seconds.

In specific application, because duration of a valid data segment in each piece of original collected data is usually less than the first preset duration threshold. Therefore, to obtain the target collected data that meets the minimum data amount needed for data analysis, the terminal device may select the at least one target valid data segment from the at least one piece of original collected data obtained by the terminal device, so that total duration of all the selected target valid data segments meets the preset duration requirement.

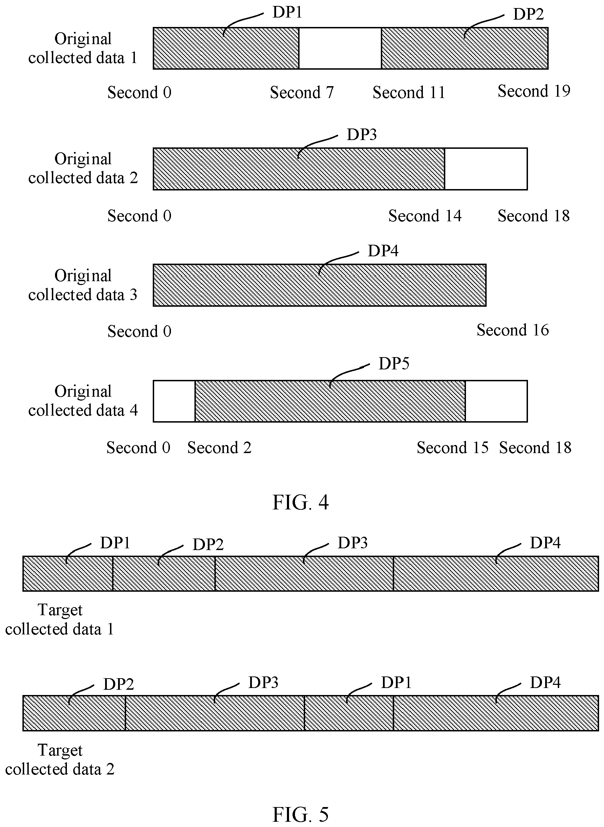

In a possible implementation of this implementation, the terminal device may sequentially select the target valid data segment from the original collected data according to a chronological order of collection time points of pieces of original collected data, until the total duration of the selected target valid data segment meets the preset duration requirement. For example, FIG. 4 shows a data structure of at least one piece of original collected data in a first preset time period. As shown in FIG. 4, it is assumed that the terminal device obtains four pieces of original collected data in a first preset time period: original collected data 1, original collected data 2, original collected data 3, and original collected data 4. Shadow parts in the original collected data indicate valid data segments, and blank parts indicate invalid data segments. To be specific, the original collected data 1 includes a valid data segment DP1 and a valid data segment DP2, where duration of DP1 is 7 seconds, and duration of DP2 is 8 seconds. The original collected data 2 includes a valid data segment DP3, where duration of DP3 is 14 seconds. The original collected data 3 includes a valid data segment DP4, where duration of DP4 is 16 seconds. The original collected data 4 includes a valid data segment DP5, where duration of DP5 is 13 seconds. It is assumed that a collection time point of the original collected data 1 is earlier than a collection time point of the original collected data 2, the collection time point of the original collected data 2 is earlier than a collection time point of the original collected data 3, and the collection time point of the original collected data 3 is earlier than a collection time point of the original collected data 4. In addition, the preset duration requirement is specifically that the duration is greater than or equal to 30 seconds. In this case, the terminal device may select DP1 and DP2 from the original collected data 1 as target valid data segments. Because total duration of DP1 and DP2 is less than 30 seconds, the terminal device may further select DP3 in the original collected data 2 as a target valid data segment. Because total duration of DP1, DP2, and DP3 is still less than 30 seconds, the terminal device may further select DP4 in the original collected data 3 as a target valid data segment. Because total duration of DP1, DP2, DP3, and DP4 is greater than 30 seconds, the terminal device stops selecting a target valid data segment in this case. Therefore, finally the selected target valid data segments are DP1, DP2, DP3, and DP4.

It may be understood that, in another possible implementation of this implementation, the terminal device may further randomly select at least one target valid data segment from the obtained at least one piece of original collected data, so that total duration of all target valid data segments meets the preset duration requirement. For example, total duration of the valid data segments DP1, DP2, and DP4 is greater than 30 seconds. Therefore, the terminal device may select the valid data segments DP1, DP2, and DP4 as the target valid data segments.

S33: Obtain the target collected data of the target user based on the target valid data segment.

In this implementation, the terminal device may splice all the target valid data segments to obtain a segment of continuous collected data, and the continuous collected data is the target collected data for subsequent data analysis.

In a possible implementation of this implementation, S33 may specifically include the following step:
splicing all target valid data segments based on collection time points of all the target valid data segments, to obtain the target collected data.

In this implementation, the terminal device may splice all the target valid data segments according to a chronological order of the collection time points of the target valid data segments, to obtain the target collected data. For example, refer to both FIG. 4 and FIG. 5. FIG. 5 shows a data structure of target collected data obtained by splicing at least one target valid data segment. Specifically, as shown in FIG. 4, the collection time point of the original collected data 1 is earlier than the collection time point of the original collected data 2, the collection time point of the original collected data 2 is earlier than the collection time point of the original collected data 3. Therefore, it can be learned that, in FIG. 4, a collection time point of DP1 is earlier than a collection time point of DP2, the collection time point of DP2 is earlier than a collection time point of DP3, the collection time point of DP3 is earlier than a collection time point of DP4. Based on this, the terminal device may splice DP1, DP2, DP3, and DP4 in a chronological order of collection time points, to obtain the target collected data 1 in FIG. 5.

In another implementation of this implementation, the terminal device may directly randomly splice all the target valid data segments without considering collection time points of the target valid data segments, to obtain the target collected data. For example, still refer to FIG. 5. The terminal device may alternatively randomly splice DP1, DP2, DP3, and DP4, to obtain the target collected data 2 in FIG. 5.

It can be learned from the foregoing that, according to the data collection method provided in this implementation, the original collected data is not discarded provided that the original collected data includes a valid data segment. In other words, a status in which the collected part of the human body is in contact with the sensor in the data collection process is not strictly limited in this implementation of this application, provided that the original collected data includes a valid data segment. This reduces a user operation requirement in the data collection process. In this implementation of this application, the at least one piece of original collected data of the target user collected by the sensor in the first preset time period is obtained; the at least one target valid data segment whose total duration meets the preset duration requirement is selected from the at least one piece of original collected data; and the target collected data of the target user is obtained based on the target valid data segment. In this way, available target collected data can be obtained based on valid data segments in a plurality of pieces of original collected data, so that a collection success rate of the available target collected data is improved and a total quantity of data collection times is reduced. Compared with the conventional technology, in this application, valid data in original collected data obtained through each collection may be used as a part of the available target collected data, so that duration of each data collection is not strictly limited and total duration of data collection is shortened.

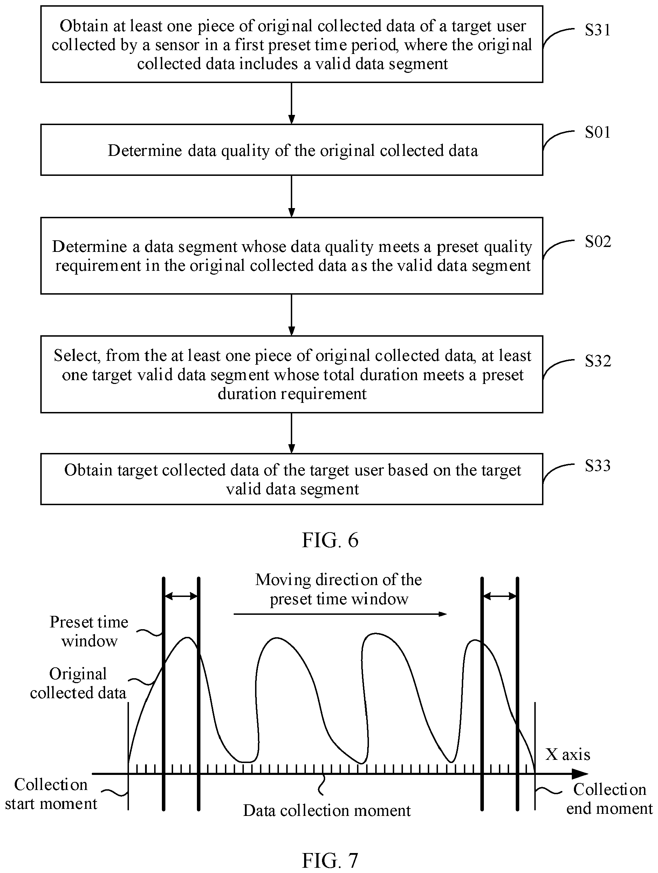

FIG. 6 is a schematic flowchart of a data collection method according to another implementation of this application. This implementation has not been claimed as such. The remaining description should be construed accordingly. Compared with the method in the implementation corresponding to FIG. 3, before S32 the data collection method provided in this implementation shown in FIG. 6 further includes S01 and S02. Details are as follows:
S01: Determine data quality of the original collected data.
S02: Determine a data segment whose data quality meets a preset quality requirement in the original collected data as the valid data segment.

In this implementation, after obtaining the at least one piece of original collected data of the target user collected in the first preset time period, the terminal device identifies a valid data segment and/or an invalid data segment included in each piece of original collected data. Specifically, the terminal device may first determine data quality of each piece of original collected data, and then identify, based on the data quality of each piece of original collected data, a valid data segment and/or an invalid data segment included in each piece of original collected data.

In a possible implementation of this implementation, when the data quality of the original collected data is determined, a preset time window and a preset step may be set. Both duration of the preset time window and the preset step may be set based on an actual requirement. For example, for a purpose of improving accuracy of data quality identification, the duration corresponding to the preset time window may be set to be relatively short. FIG. 7 shows a schematic waveform of a piece of original collected data collected by a sensor. As shown in FIG. 7, each small scale on an X axis represents a data collection moment. The terminal device may slide a preset time window based on a preset step from a collection start moment of the original collected data to a collection end moment of the original collected data. Each time the terminal device slides the preset time window, the terminal device determines data quality of a data segment in a data collection time period corresponding to the preset time window. In specific application, the terminal device may determine an average value of data quality at all data collection moments in the data collection time period in which the preset time window stays each time as the data quality of the data segments in the data collection time period in which the preset time window stays. After the terminal device determines the data quality of the data segments in the data collection time period corresponding to the preset time window after each sliding of the preset time window, the terminal device may identify a data segment whose data quality meets the preset quality requirement as a valid data segment, and identify a data segment whose data quality does not meet the preset quality requirement as an invalid data segment.

It should be noted that the data quality of the original collected data is specifically described through a preset data feature of the to-be-detected data corresponding to the original collected data, and the preset data feature may be determined based on a type of the to-be-detected data.

In this implementation, the original collected data is divided, based on the preset time window and the preset step, into a plurality of data segments that can be overlapped. Data quality of each data segment is determined, and a valid data segment and/or an invalid data segment included in the original collected data is identified based on the data quality of each data segment, so that accuracy of identifying the valid data segment and/or the invalid data segment in the original collected data can be improved.

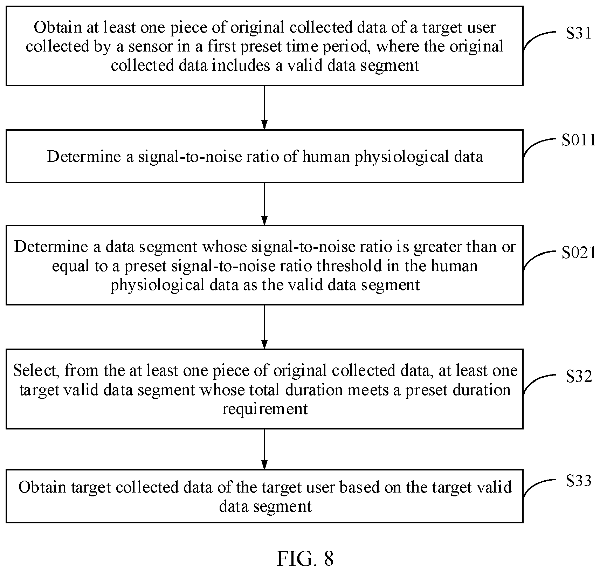

In a possible implementation of this implementation, when the preset physiological feature of the human body needs to be detected, the original collected data may include the human physiological data that can represent the preset physiological feature. For example, when the activity feature of the human heart needs to be detected, the original collected data may include the electrocardio signal that can represent the activity feature of the human heart. In this case, the preset data feature may be the signal-to-noise ratio. In other words, the data quality of the human physiological data may be described using the signal-to-noise ratio of the human physiological data. Based on this, S01 may be specifically implemented using S011 shown in FIG. 8. This implementation has not been claimed as such. The remaining description should be construed accordingly. Correspondingly, S02 may specifically include S021. Details are as follows:
S011: Determine a signal-to-noise ratio of the human physiological data.
S021: Determine a data segment whose signal-to-noise ratio is greater than or equal to a preset signal-to-noise ratio threshold in the human physiological data as the valid data segment.

In this implementation, when identifying a valid data segment and/or an invalid data segment in each piece of human physiological data, the terminal device may first determine a signal-to-noise ratio of each piece of human physiological data, and then identify the valid data segment and/or the invalid data segment in each piece of human physiological data based on the signal-to-noise ratio of each piece of human physiological data.

In a possible implementation of this implementation, when the signal-to-noise ratio of each piece of human physiological data is determined, a preset time window and a preset step may be set. Both duration of the preset time window and the preset step may be set based on an actual requirement. For example, for a purpose of improving accuracy of signal-to-noise ratio identification, the duration corresponding to the preset time window may be set to be relatively short. Still refer to FIG. 7. In this implementation, the original collected data in FIG. 7 is the preset human physiological data. As shown in FIG. 7, each small scale on an X axis represents a data collection moment. The terminal device may slide a preset time window based on a preset step from a collection start moment of the human physiological data to a collection end moment of the human physiological data. Each time the terminal device slides the preset time window, the terminal device determines a signal-to-noise ratio of a data segment in a data collection time period corresponding to the preset time window. In specific application, the terminal device may determine an average value of signal-to-noise ratios at all data collection moments in the data collection time period in which the preset time window stays each time as the signal-to-noise ratios of the data segments in the data collection time period in which the preset time window stays each time. After the terminal device determines the signal-to-noise ratios of the data segments in the data collection time period corresponding to the preset time window after each sliding of the preset time window, the terminal device may identify a data segment whose signal-to-noise ratio meets the preset quality requirement as a valid data segment in the human physiological data, and identify a data segment whose signal-to-noise ratio does not meet the preset quality requirement as an invalid data segment in the human physiological data.

In this implementation, when the original collected data includes the preset human physiological data, the valid data segment and/or the invalid data segment in the human physiological data are/is identified directly based on the signal-to-noise ratio of the human physiological data. This improves efficiency of identifying the valid data segment and/or the invalid data segment.

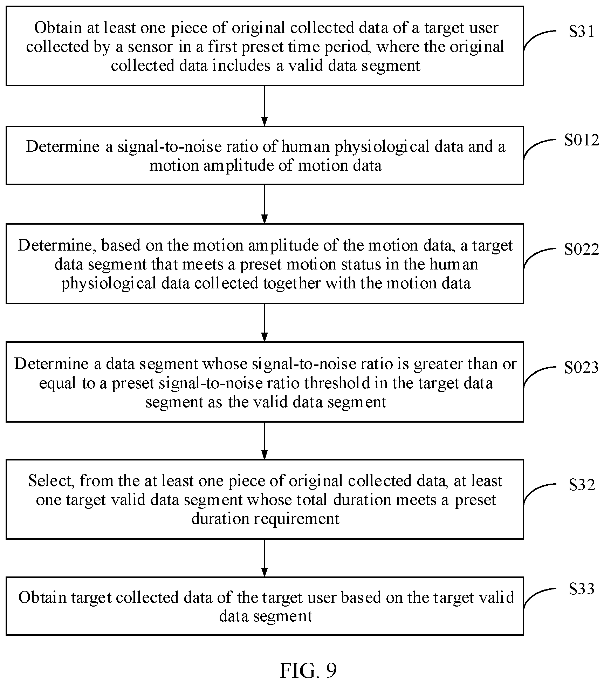

In another possible implementation of this implementation, when the preset human physiological data is collected, a motion status of the collected part of the human body affects data quality of the human physiological data collected by the sensor. Therefore, for a purpose of improving accuracy of data quality recognition, when collecting the preset human physiological data, the wearable device may further collect the motion data of the collected part of the human body. In other words, in addition to the preset human physiological data, the original collected data may further include the motion data that is collected together with the preset human physiological data and that can represent the motion status of the collected part of the human body. For example, when the activity feature of the human heart needs to be detected, in addition to the electrocardio signal that can represent the activity feature of the human heart, the original collected data may further include an acceleration that is collected together with the electrocardio signal of the human body and that can represent the motion status of the collected part of the human body. Specifically, in this implementation, when controlling the sensor that is configured to collect the preset human physiological data to collect preset human physiological data of the user, the wearable device further controls a motion sensor to collect the motion data of the collected part of the user. It may be understood that a collection time point of each piece of human physiological data is the same as a collection time point of motion data that is of the collected part of the human body and that is collected together with the human physiological data, and duration of each piece of human physiological data is the same as duration of the motion data that is of the collected part of the human body and that is collected together with the human physiological data. The wearable device may associate the human physiological data with the motion data of the collected part of the human body that are collected together. The terminal device may store, in the local memory of the terminal device, the association between each piece of human physiological data of each user and motion data that is of a collected part of a human body and that is collected together with the human physiological data. In this case, the terminal device may identify the valid data segment and/or the invalid data segment in the human physiological data based on the signal-to-noise ratio of the human physiological data and an amplitude of the motion data of the collected part of the human body. Based on this, S01 is specifically implemented using S012 shown in FIG. 9. This implementation is according to the claimed invention. Correspondingly, S02 may specifically include S022 and S023. Details are as follows:
S012: Determine a signal-to-noise ratio of the human physiological data and a motion amplitude of the motion data.
S022: Determine, based on the motion amplitude of the motion data, a target data segment that meets a preset motion status in the human physiological data collected together with the motion data.
S023: Determine a data segment whose signal-to-noise ratio is greater than or equal to a preset signal-to-noise ratio threshold in the target data segment as the valid data segment.

In this implementation, when the terminal device identifies the valid data segment and/or the invalid data segment in the human physiological data based on the signal-to-noise ratio of the human physiological data and the motion amplitude of the motion data that is of the collected part of the human body that is collected together with the human physiological data, the terminal device may first determine the signal-to-noise ratio of each piece of human physiological data and the motion amplitude of motion data that is collected together with each piece of human physiological data.

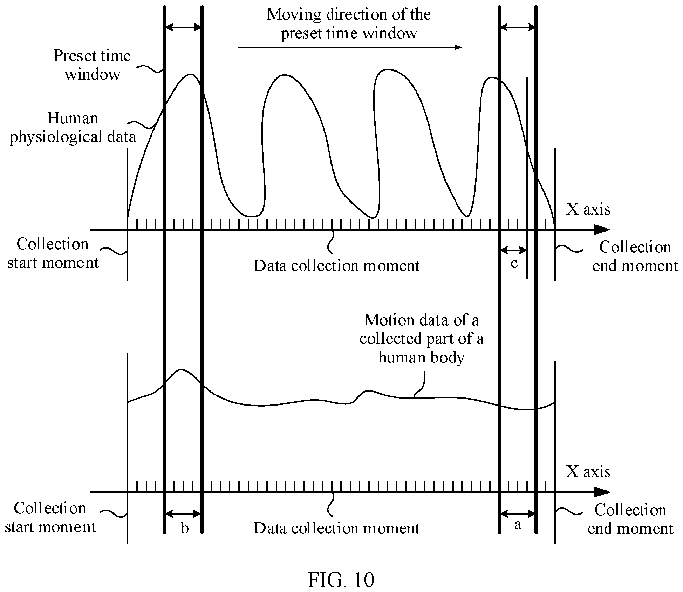

In the context of the claimed invention, both the signal-to-noise ratio of each piece of human physiological data and the motion amplitude of motion data that is collected together with each piece of human physiological data are determined, a preset time window and a preset step may be set. Both duration of the preset time window and the preset step may be set based on an actual requirement. For example, for a purpose of improving accuracy of signal-to-noise ratio identification, the duration corresponding to the preset time window may be set to be relatively short. FIG. 10 shows a schematic waveform of preset human physiological data and a schematic waveform of motion data that is of a collected part of a human body and that is collected together with the preset human physiological data according to an implementation of this application. As shown in FIG. 10, each small scale on an X axis represents a data collection moment. The terminal device may slide a preset time window based on a preset step from a collection start moment of the human physiological data and the motion data to a collection end moment of the human physiological data and the motion data. Each time the terminal device slides the preset time window, the terminal device determines a signal-to-noise ratio of a data segment corresponding to the human physiological data in a data collection time period in which the preset time window stays each time, and an amplitude of a data segment corresponding to the motion data in the data collection time period in which the preset time window stays each time. In specific application, the terminal device may determine an average value of signal-to-noise ratios of the human physiological data corresponding to all data collection moments in the data collection time period in which each preset time window stays as the signal-to-noise ratio of the data segment corresponding to the human physiological data in the data collection time period in which the preset time window stays, and determine an average value of amplitudes of motion data corresponding to all the data collection moments in the data collection time period in which the preset time window stays as the amplitude of the data segment corresponding to the motion data in the data collection time period in which the preset time window stays.

After the terminal device determines, after each sliding of the preset time window, the amplitude of the motion data and the signal-to-noise ratio of the human physiological data in the data collection time period in which the preset time window stays, the terminal device may determine, after each sliding of the preset time window based on the amplitude of the motion data in the data collection time period in which the preset time window stays, the target data segment that meets the preset motion status in the corresponding human physiological data. Specifically, the terminal device may determine a data segment that is in each piece of motion data and whose motion amplitude is less than or equal to a preset amplitude threshold as a first reference data segment, and determine a data segment that is in the human physiological data corresponding to each piece of motion data and that is in the same time period with the first reference data segment as the target data segment that meets the preset motion status. For example, still refer to FIG. 10. It is assumed that an amplitude of motion data of a collected part of a human body in a data collection time period a in which the preset time window stays is less than or equal to the preset amplitude threshold, the terminal device determines a data segment that is in the motion data and that is in the data collection time period a as the first reference data segment. Correspondingly, the terminal device determines a data segment that is in the human physiological data corresponding to the motion data and that is in the data collection time period a as the target data segment.

After determining the target data segment in each piece of human physiological data, the terminal device determines a data segment, in the target data segment, whose signal-to-noise ratio is greater than or equal to the preset signal-to-noise ratio threshold as the valid data segment. For example, still refer to FIG. 10. It is assumed that in a data segment that is in the human physiological data and that is in the data collection time period a, only a signal-to-noise ratio of a data segment that is in a collection time period c is greater than or equal to the preset signal-to-noise ratio threshold, the terminal device determines the data segment that is in the human physiological data and that is in the collection time period c as the valid data segment.

In the context of the claimed invention, the valid data segment and/or the invalid data segment in the human physiological data are/is identified based on a combination of the amplitude of the motion data of the collected part of the human body and the signal-to-noise ratio of the human physiological data, so that accuracy of identifying the valid data segment and/or the invalid data segment in the human physiological data can be improved. In this way, the finally obtained target collected data can more accurately reflect a corresponding human physiological feature of the user.

In another implementation of this application, alternatively, the valid data segment and/or the invalid data segment in the human physiological data corresponding to the motion data may be identified based only on the motion data of the collected part of the human body. Based on this, after determining the target data segment in the corresponding human physiological data based on each piece of motion data, the terminal device directly identifies the target data segment in each piece of human physiological data as the valid data segment of each piece of human physiological data.

In still another implementation of this application, the terminal device may determine a data segment that is in each piece of motion data and whose motion amplitude is greater than a preset amplitude threshold as a second reference data segment, and determine a data segment that is in the human physiological data corresponding to each piece of motion data and that is in the same time period with the second reference data segment as the invalid data segment. For example, still refer to FIG. 10. It is assumed that an amplitude of motion data of a collected part of a human body in a data collection time period b in which the preset time window stays is greater than the preset amplitude threshold, the terminal device determines a data segment that is in the motion data and that is in the data collection time period b as the second reference data segment. Correspondingly, the terminal device determines a data segment that is in the human physiological data corresponding to the motion data and that is in the data collection time period b as the invalid data segment.

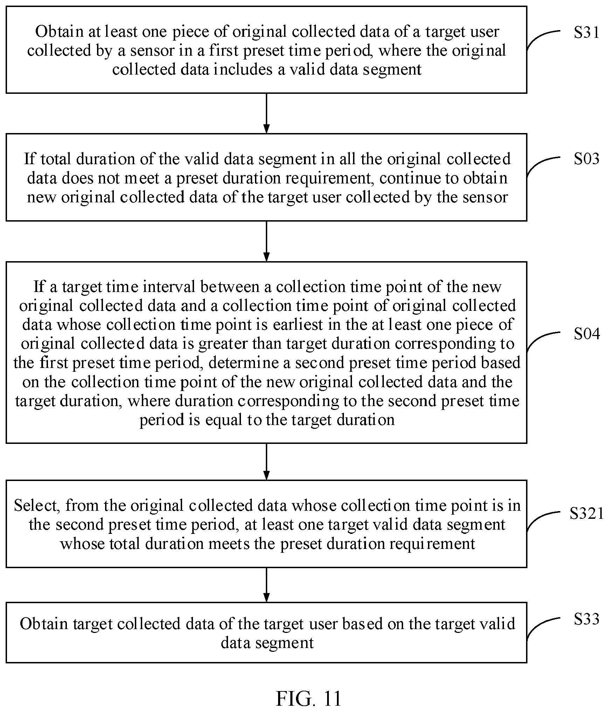

FIG. 11 is a schematic flowchart of a data collection method according to another implementation of this application. Compared with the method in the implementation corresponding to FIG. 3, after S31 and before S32, the data collection method provided in this implementation shown in FIG. 11 further includes S03 and S04. Details are as follows:
S03: If total duration of the valid data segment in all the original collected data does not meet the preset duration requirement, continue to obtain new original collected data of the target user collected by the sensor.
S04: If a target time interval between a collection time point of the new original collected data and a collection time point of original collected data whose collection time point is earliest in the at least one piece of original collected data is greater than the target duration corresponding to the first preset time period, determine a second preset time period based on the collection time point of the new original collected data and the target duration, where duration corresponding to the second preset time period is equal to the target duration.

Correspondingly, S32 may specifically include S321. Details are as follows:
S321: Select, from the original collected data whose collection time point is in the second preset time period, the at least one target valid data segment whose total duration meets the preset duration requirement.

In this implementation, the terminal device obtains only the at least one piece of original collected data of the target user collected by the sensor in the first preset time period each time. When total duration of all valid data segments in the at least one piece of original collected data does not meet the preset duration requirement, for example, when the total duration of all the valid data segments in the at least one piece of original collected data is less than the first preset duration threshold, to ensure that the duration of the target collected data can meet the requirement, the terminal device may continue to obtain the new original collected data of the target user collected by the sensor. The new original collected data refers to original collected data whose collection time is later than a collection time point of all the original collected data in the at least one piece of original collected data.

In a possible implementation of this implementation, if the memory of the terminal device does not store the new original collected data of the target user, the terminal device may control the sensor in the wearable device to continue to collect to-be-detected data of the target user. The to-be-detected data of the target user that is continuously collected by the sensor is the new original collected data of the target user. The terminal device obtains the new original collected data.

In another possible implementation of this implementation, if the memory of the terminal device stores the new original collected data of the target user, the terminal device directly obtains the new original collected data of the target user from the memory of the terminal device.

For a purpose of ensuring that in the original collected data used by the terminal device to determine the target collected data, a time interval between the collection time point of the original collected data whose collection time point is earliest and the collection time point of the original collected data whose collection time point is latest is less than or equal to the target duration corresponding to the first preset time period, after the terminal device obtains the new original collected data of the target user, the terminal device calculates the target time interval between the collection time point of the new original collected data and the collection time point of the original collected data whose collection time point is earliest in the at least one piece of original collected data, and compares the target time interval with the target duration corresponding to the first preset time period.

In this implementation, if the terminal device detects that the target time interval is greater than the target duration corresponding to the first preset time period, the terminal device determines the second preset time period based on the collection time point of the new original collected data and the target duration. The duration corresponding to the second preset time period is the same as the target duration, and a collection time point of each piece of original collected data in the second preset time period is not totally the same as a collection time point of each piece of original collected data in the first preset time period.

After determining the second preset time period, the terminal device selects, from the original collected data whose collection time point is in the second preset time period, the at least one target valid data segment whose total duration meets the preset duration requirement. It should be noted that, for a specific manner in which the terminal device selects the target valid data segment from the at least one piece of original collected data in the second preset time period, refer to a manner in which the terminal device selects the target valid data segment from the at least one piece of original collected data in the first preset time period. Details are not described herein again.

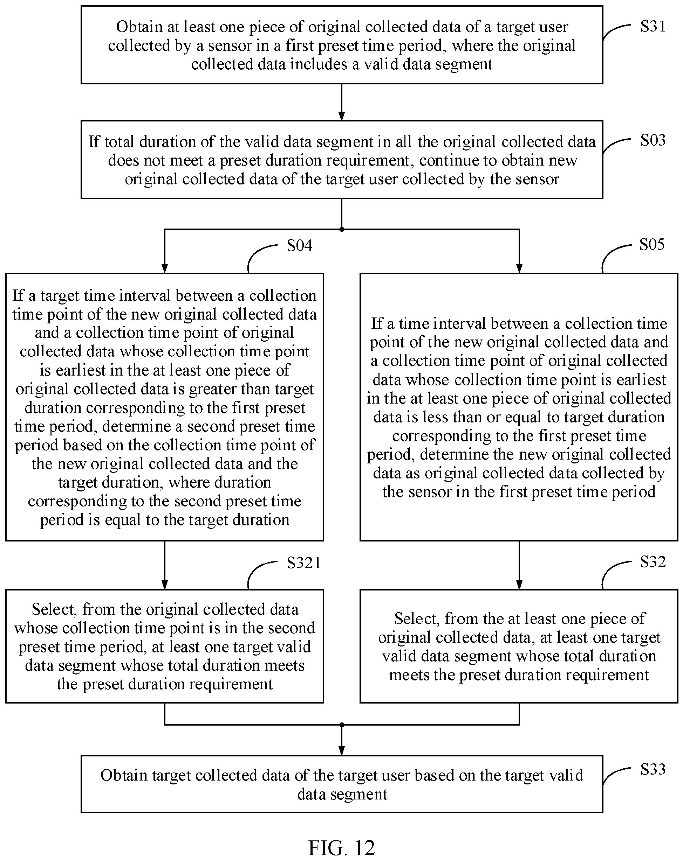

In still another implementation of this application, if the terminal device detects that the target time interval is less than or equal to the target duration corresponding to the first preset time period, the terminal device may perform S05 shown in FIG. 12. Details are as follows:
S05: If the target time interval between the collection time point of the new original collected data and the collection time point of the original collected data whose collection time point is earliest in the at least one piece of original collected data is less than or equal to the target duration corresponding to the first preset time period, determine the new original collected data as original collected data collected by the sensor in the first preset time period.

In this implementation, when the terminal device detects that the target time interval is less than or equal to the target duration corresponding to the first preset time period, it indicates that the collection time point of the new original collected data is in the first preset time period. In this case, the terminal device determines the new original collected data as the original collected data of the target user collected by the sensor in the first preset time period, and further determines the target collected data of the target user based on the original collected data of the target user collected by the sensor in the first preset time period.

In this implementation, while it is ensured that the duration of the target collected data meets the requirement, in all the original collected data used when the terminal device determines the target collected data, the time interval between the collection time point of the original collected data whose collection time point is earliest and the collection time point of the original collected data whose collection time point is latest is enabled to be less than the target duration corresponding to the first preset time period. This can improve accuracy of subsequent data analysis.

It may be understood that sequence numbers of the processes do not mean execution sequences in the foregoing implementations. The execution sequences of the processes should be determined according to functions and internal logic of the processes, and should not be construed as any limitation on the implementation processes of implementations of this application.

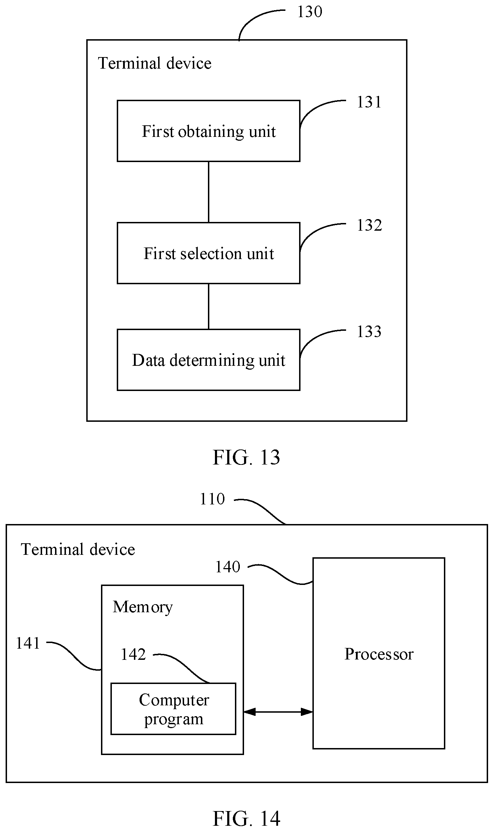

Corresponding to the data collection method in the foregoing implementations, FIG. 13 is a block diagram of a structure of a terminal device according to an implementation of this application. Units included in the terminal device are configured to perform steps in the foregoing implementations. For details, refer to related descriptions in the foregoing implementations. For ease of description, only a part related to implementations of this application is shown. In actual application, the terminal device may be a wearable device, or may be a mobile terminal such as a mobile phone or a tablet computer. As shown in FIG. 13, the terminal device 130 includes a first obtaining unit 131, a first selection unit 132, and a data determining unit 133.

The first obtaining unit 131 is configured to obtain at least one piece of original collected data of a target user collected by a sensor in a first preset time period, where the original collected data includes a valid data segment.

The first selection unit 132 is configured to select, from the at least one piece of original collected data, at least one target valid data segment whose total duration meets a preset duration requirement.

The data determining unit 133 is configured to obtain target collected data of the target user based on the target valid data segment.

In an implementation of this application, the terminal device 130 further includes a second determining unit and a third determining unit.

The second determining unit is configured to determine data quality of the original collected data.

The third determining unit is configured to determine a data segment whose data quality meets a preset quality requirement in the original collected data as the valid data segment.

In an implementation of this application, the original collected data includes preset human physiological data.

The second determining unit is specifically configured to determine a signal-to-noise ratio of the human physiological data.

Correspondingly, the third determining unit is specifically configured to determine a data segment whose signal-to-noise ratio is greater than or equal to a preset signal-to-noise ratio threshold in the human physiological data as the valid data segment.

In the context of the claimed invention, the original collected data includes preset human physiological data and motion data that is of a collected part of a human body and that is collected together with the human physiological data.

The second determining unit is specifically configured to determine a signal-to-noise ratio of the human physiological data and a motion amplitude of the motion data.

Correspondingly, the third determining unit is specifically configured to: determine, based on the motion amplitude of the motion data, a target data segment that meets a preset motion status in the human physiological data collected together with the motion data; and determine a data segment whose signal-to-noise ratio is greater than or equal to the preset signal-to-noise ratio threshold in the target data segment as the valid data segment.

In an implementation of this application, the first obtaining unit 131 is further configured to: if total duration of the valid data segment in all the original collected data does not meet the preset duration requirement, continue to obtain new original collected data of the target user collected by the sensor. The terminal device 130 further includes a fourth determining unit.

The fourth determining unit is configured to: if a target time interval between a collection time point of the new original collected data and a collection time point of original collected data whose collection time point is earliest in the at least one piece of original collected data is greater than target duration corresponding to the first preset time period, determine a second preset time period based on the collection time point of the new original collected data and the target duration, where duration corresponding to the second preset time period is equal to the target duration.

Correspondingly, the first selection unit 132 is further configured to select, from the original collected data whose collection time point is in the second preset time period, at least one target valid data segment whose total duration meets the preset duration requirement.

In an implementation of this application, the terminal device 130 further includes a fifth determining unit.

The fifth determining unit is configured to: if the target time interval between the collection time point of the new original collected data and the collection time point of the original collected data whose collection time point is earliest in the at least one piece of original collected data is less than or equal to the target duration corresponding to the first preset time period, determine the new original collected data as original collected data collected by the sensor in the first preset time period.

In an implementation of this application, the data determining unit 133 is specifically configured to splice all target valid data segments based on collection time points of all the target valid data segments, to obtain the target collected data.

It can be learned from the foregoing that, according to the terminal device provided in this implementation of this application, the original collected data is not discarded provided that the original collected data includes a valid data segment. In other words, a status in which the collected part of the human body is in contact with the sensor in the data collection process is not strictly limited in this implementation of this application, provided that the original collected data includes a valid data segment. This reduces a user operation requirement in the data collection process. In this implementation of this application, the at least one piece of original collected data of the target user collected by the sensor in the first preset time period is obtained; the at least one target valid data segment whose total duration meets the preset duration requirement is selected from the at least one piece of original collected data; and the target collected data of the target user is obtained based on the target valid data segment. In this way, available target collected data can be obtained based on valid data segments in a plurality of pieces of original collected data, so that a collection success rate of the available target collected data is improved and a total quantity of data collection times is reduced. Compared with the conventional technology, in this application, valid data in original collected data obtained through each collection may be used as a part of the available target collected data, so that duration of each data collection is not strictly limited and total duration of data collection is shortened.

FIG. 14 is a schematic diagram of a structure of a terminal device according to another implementation of this application. As shown in FIG. 14, the terminal device 110 in this implementation includes: at least one processor 140 (only one processor is shown in FIG. 14), a memory 141, and a computer program 142 that is stored in the memory 141 and that runs on the at least one processor 140. When executing the computer program 142, the processor 140 implements steps in any one of the foregoing data collection method implementations.

The terminal device 110 may be a computing device such as a desktop computer, a notebook computer, a palmtop computer, or a cloud server. The terminal device may include but is not limited to the processor 140 and the memory 141. Persons skilled in the art may understand that FIG. 14 is merely an example of the terminal device 110, and does not constitute any limitation on the terminal device 110. The terminal device may include more or fewer components than those shown in the figure, or some components may be combined, or different components may be used. For example, the terminal device may further include an input/output device, a network access device, or the like.

The processor 140 may be a central processing unit (Central Processing Unit, CPU). The processor 140 may alternatively be another general-purpose processor, a digital signal processor (Digital Signal Processor, DSP), an application-specific integrated circuit (Application-Specific Integrated Circuit, ASIC), a field-programmable gate array (Field-Programmable Gate Array, FPGA) or another programmable logic device, a discrete gate or transistor logic device, a discrete hardware component, or the like. The general-purpose processor may be a microprocessor, or the processor may be any conventional processor or the like.

In some implementations, the memory 141 may be an internal storage unit of the terminal device 110, for example, a hard disk drive or an internal storage of the terminal device 110. In some other implementations, the memory 141 may alternatively be an external storage device of the terminal device 110, for example, a removable hard disk, a smart media card (Smart Media Card, SMC), a secure digital (Secure Digital, SD) card, a flash card (Flash Card), or the like that is configured on the terminal device 110. Further, the memory 141 may alternatively include both the internal storage unit and the external storage device of the terminal device 110. The memory 141 is configured to store an operating system, an application, a bootloader (BootLoader), data, and another program, for example, program code of the computer program. The memory 141 may further be configured to temporarily store output data or to-be-output data.

It should be noted that content such as information exchange between the foregoing apparatuses/units and the execution processes thereof is based on a same concept as the method implementations of this application. For specific functions and technical effects of the content, refer to the method implementations. Details are not described herein again.

Persons skilled in the art may clearly understand that, for the purpose of convenient and brief description, division into the foregoing functional units or modules is merely used as an example for description. In actual application, the foregoing functions may be allocated to different functional units or modules for implementation according to a requirement. That is, an inner structure of the apparatus is divided into different functional units or modules to implement all or some of the functions described above. Functional units and modules in implementations may be integrated into one processing unit, or each of the units may exist alone physically, or two or more units may be integrated into one unit. The integrated unit may be implemented in a form of hardware, or may be implemented in a form of a software functional unit. In addition, specific names of the functional units or modules are merely provided for the purpose of distinguishing the units and modules from one another, but are not intended to limit the protection scope of this application. For a specific working process of the units and modules in the foregoing system, refer to a corresponding process in the foregoing method implementations. Details are not described herein again.

An implementation of this application further provides a computer-readable storage medium. The computer-readable storage medium stores a computer program. When the computer program is executed by a processor, the steps in the foregoing data collection method may be implemented.

An implementation of this application provides a computer program product. When the computer program product runs on a mobile terminal, the mobile terminal is enabled to implement the steps in the foregoing data collection method when executing the computer program product.

When the integrated unit is implemented in the form of the software functional unit and sold or used as an independent product, the integrated unit may be stored in a computer-readable storage medium. Based on such an understanding, in this application, all or some of the procedures of the methods in the foregoing implementations may be implemented by a computer program instructing related hardware. The computer program may be stored in a computer-readable storage medium. When the computer program is executed by a processor, the steps in the foregoing method implementations can be implemented. The computer program includes computer program code, and the computer program code may be in a source code form, an object code form, an executable file form, some intermediate forms, or the like. The computer-readable medium may include at least any entity or apparatus capable of carrying the computer program code to an apparatus/terminal device, a recording medium, a computer memory, a read-only memory (ROM, Read-Only Memory), a random access memory (RAM, Random Access Memory), an electrical carrier signal, a telecommunications signal, and a software distribution medium, for example, a USB flash drive, a removable hard disk, a magnetic disk, or an optical disc. In some jurisdictions, according to legislation and patent practice, a computer-readable medium cannot be an electrical carrier signal or a telecommunications signal.

In the foregoing implementations, the description of each implementation has respective focuses. For a part that is not described in detail in an implementation, refer to related descriptions in other implementations.

Persons of ordinary skill in the art may be aware that, in combination with the examples described in implementations disclosed in this specification, units and algorithm steps may be implemented by electronic hardware or a combination of computer software and electronic hardware. Whether the functions are performed by hardware or software depends on particular applications and design constraint conditions of the technical solutions. Persons skilled in the art may use different methods to implement the described functions for each particular application, but it should not be considered that the implementation goes beyond the scope of this application.

In implementations provided in this application, it should be understood that the disclosed apparatus/network device and method may be implemented in other manners. For example, the described apparatus/network device implementation is merely an example. For example, the module or unit division is merely logical function division and may be other division in actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electronic, mechanical, or other forms.

The units described as separate parts may or may not be physically separate, and parts displayed as units may or may not be physical units, may be located in one position, or may be distributed on a plurality of network units. Some or all of the units may be selected according to actual requirements to achieve the objectives of the solutions of implementations.

The scope of protection is defined by the appended claims.

## Claims

1. A computer-implemented data collection method, comprising:
obtaining (S31) at least one piece of original collected data of a target user collected by a sensor in a first preset time period, wherein the original collected data comprises a valid data segment;
selecting (S32), from the at least one piece of original collected data, one or more target valid data segments whose total duration meets a preset duration requirement; and
obtaining (S33) target collected data of the target user based on the one or more target valid data segments;
wherein before the selecting, from the at least one piece of original collected data, one or more target valid data segments whose total duration meets a preset duration requirement, the method further comprises:
determining data quality of the original collected data; and
determining a data segment whose data quality meets a preset quality requirement in the original collected data as the valid data segment;
**characterised in that**
the original collected data comprises preset human physiological data and motion data that is of a collected part of a human body and that is collected together with the human physiological data; and the determining data quality of the original collected data comprises:
determining a signal-to-noise ratio of the human physiological data and a motion amplitude of the motion data; and
the determining a data segment whose data quality meets a preset quality requirement in the original collected data as the valid data segment comprises:
determining, based on the motion amplitude of the motion data, a target data segment that meets a preset motion status in the human physiological data collected together with the motion data, wherein the target data segment meets the preset motion status when motion amplitude of each piece of motion data in the target data segment is less than or equal to a preset amplitude threshold; and
determining a data segment whose signal-to-noise ratio is greater than or equal to a preset signal-to-noise ratio threshold in the target data segment as the valid data segment.

2. The data collection method according to claim 1, wherein after the obtaining at least one piece of original collected data of a target user collected by a sensor in a first preset time period, and before the selecting, from the at least one piece of original collected data, one or more target valid data segments whose total duration meets a preset duration requirement, the method further comprises:
if total duration of the one or more valid data segments in all the original collected data does not meet the preset duration requirement, continuing to obtain new original collected data of the target user collected by the sensor; and
if a target time interval between a collection time point of the new original collected data and a collection time point of original collected data whose collection time point is earliest in the at least one piece of original collected data is greater than target duration corresponding to the first preset time period, determining a second preset time period based on the collection time point of the new original collected data and the target duration, wherein duration corresponding to the second preset time period is equal to the target duration; and
the selecting, from the at least one piece of original collected data, one or more target valid data segments whose total duration meets a preset duration requirement comprises:
selecting, from the original collected data whose collection time point is in the second preset time period, the one or more target valid data segments whose total duration meets the preset duration requirement.

3. The data collection method according to claim 2, wherein after the continuing to obtain new original collected data of the target user collected by the sensor if total duration of the valid data segment in all the original collected data does not meet the preset duration requirement, and before the selecting, from the original collected data whose collection time point is in the second preset time period, the one or more target valid data segments whose total duration meets the preset duration requirement, the method further comprises:
if the target time interval between the collection time point of the new original collected data and the collection time point of the original collected data whose collection time point is earliest in the at least one piece of original collected data is less than or equal to the target duration corresponding to the first preset time period, determining the new original collected data as original collected data collected by the sensor in the first preset time period.

4. The data collection method according to any one of claims 1 to 3, wherein the obtaining target collected data of the target user based on the one or more target valid data segment comprises:
splicing all target valid data segments based on collection time points of all the target valid data segments, to obtain the target collected data.

5. A terminal device (100), comprising a memory (120), a processor (180), and a computer program that is stored in the memory and that can be run on the processor, wherein when executing the computer program, the processor implements the data collection method according to any one of claims 1 to 4.

6. A computer-readable storage medium, wherein the computer-readable storage medium stores a computer program, and when the computer program is executed by a processor, the data collection method according to any one of claims 1 to 4 is implemented.

## Patentansprüche

1. Computerimplementiertes Datenerfassungsverfahren, umfassend:
Erhalten (S31) mindestens eines Teils der ursprünglich erfassten Daten eines Zielbenutzers, die von einem Sensor in einem ersten voreingestellten Zeitraum erfasst wurden, wobei die ursprünglich erfassten Daten ein gültiges Datensegment umfassen;
Auswählen (S32) aus dem mindestens einen Teil der ursprünglich erfassten Daten eines oder mehrerer gültiger Zieldatensegmente, deren Gesamtdauer eine voreingestellte Daueranforderung erfüllt; und
Erhalten (S33) der erfassten Zieldaten des Zielbenutzers auf der Grundlage des einen oder der mehreren gültigen Zieldatensegmente;
wobei vor dem Auswählen eines oder mehrerer gültiger Zieldatensegmente, deren Gesamtdauer eine voreingestellte Daueranforderung erfüllt, aus dem mindestens einen Teil der ursprünglich erfassten Daten das Verfahren ferner Folgendes umfasst:
Bestimmen der Datenqualität der ursprünglich erfassten Daten; und
Bestimmen eines Datensegments, dessen Datenqualität eine voreingestellte Qualitätsanforderung in den ursprünglich erfassten Daten erfüllt, als gültiges Datensegment;
**dadurch gekennzeichnet, dass**
die ursprünglich erfassten Daten voreingestellte menschliche physiologische Daten und Bewegungsdaten umfassen, die von einem erfassten Teil eines menschlichen Körpers stammen und die zusammen mit den menschlichen physiologischen Daten erfasst werden; und die bestimmende Datenqualität der ursprünglich erfassten Daten Folgendes umfasst:
Bestimmen eines Signal-Rausch-Verhältnisses der menschlichen physiologischen Daten und einer Bewegungsamplitude der Bewegungsdaten; und
das Bestimmen eines Datensegments, dessen Datenqualität eine voreingestellte Qualitätsanforderung in den ursprünglich erfassten Daten erfüllt, als gültiges Datensegment Folgendes umfasst:
Bestimmen, basierend auf der Bewegungsamplitude der Bewegungsdaten, eines Zieldatensegments, das einen voreingestellten Bewegungsstatus in den zusammen mit den Bewegungsdaten erfassten menschlichen physiologischen Daten erfüllt, wobei das Zieldatensegment den voreingestellten Bewegungsstatus erfüllt, wenn die Bewegungsamplitude jedes Bewegungsdatenelements im Zieldatensegment kleiner oder gleich einem voreingestellten Amplitudenschwellenwert ist; und
Bestimmen eines Datensegments, dessen Signal-Rausch-Verhältnis größer oder gleich einem voreingestellten Signal-Rausch-Verhältnis-Schwellenwert im Zieldatensegment ist, als gültiges Datensegment.

2. Datenerfassungsverfahren nach Anspruch 1, wobei nach dem Erhalten von mindestens einem Teil der ursprünglich erfassten Daten eines Zielbenutzers, die von einem Sensor in einem ersten voreingestellten Zeitraum erfasst wurden, und vor dem Auswählen eines oder mehrerer gültiger Zieldatensegmente aus dem mindestens einen Teil der ursprünglich erfassten Daten, deren Gesamtdauer eine voreingestellte Daueranforderung erfüllt, das Verfahren ferner Folgendes umfasst:
wenn die Gesamtdauer des einen oder der mehreren gültigen Datensegmente in allen ursprünglich erfassten Daten die voreingestellte Daueranforderung nicht erfüllt, weiterhin Erhalten neuer, ursprünglich erfasster Daten des Zielbenutzers, die vom Sensor erfasst wurden; und
wenn ein Zielzeitintervall zwischen einem Erfassungszeitpunkt der neuen ursprünglich erfassten Daten und einem Erfassungszeitpunkt der ursprünglich erfassten Daten, dessen Erfassungszeitpunkt in dem mindestens einen Teil der ursprünglich erfassten Daten am frühesten liegt, größer als die Zieldauer ist, die dem ersten voreingestellten Zeitraum entspricht, Bestimmen eines zweiten voreingestellten Zeitraums basierend auf dem Erfassungszeitpunkt der neuen ursprünglich erfassten Daten und der Zieldauer, wobei die Dauer, die dem zweiten voreingestellten Zeitraum entspricht, gleich der Zieldauer ist; und
das Auswählen eines oder mehrerer gültiger Zieldatensegmente, deren Gesamtdauer einer voreingestellten Daueranforderung entspricht, aus dem mindestens einen Teil der ursprünglich erfassten Daten Folgendes umfasst:
Auswählen des einen oder der mehreren gültigen Zieldatensegmente, deren Gesamtdauer die voreingestellte Daueranforderung erfüllt, aus den ursprünglich erfassten Daten,
deren Erfassungszeitpunkt in dem zweiten voreingestellten Zeitraum liegt.

3. Datenerfassungsverfahren nach Anspruch 2, wobei nach dem Fortfahren mit dem Erhalten neuer ursprünglich erfasster Daten des Zielbenutzers, die durch den Sensor erfasst wurden, die Gesamtdauer des gültigen Datensegments in allen ursprünglich erfassten Daten die voreingestellte Daueranforderung nicht erfüllt und vor dem Auswählen des einen oder der mehreren gültigen Zieldatensegmente, deren Gesamtdauer die voreingestellte Daueranforderung erfüllt, aus den ursprünglich erfassten Daten, deren Erfassungszeitpunkt in der zweiten voreingestellten Zeitspanne liegt, das Verfahren ferner Folgendes umfasst:
wenn das Zielzeitintervall zwischen dem Erfassungszeitpunkt der neuen ursprünglich erfassten Daten und dem Erfassungszeitpunkt der ursprünglich erfassten Daten, deren Erfassungszeitpunkt in dem mindestens einen Teil der ursprünglich erfassten Daten am frühesten liegt, kleiner oder gleich der Zieldauer ist, die dem ersten voreingestellten Zeitraum entspricht, Bestimmen der neuen ursprünglich erfassten Daten als ursprünglich erfasste Daten, die von dem Sensor in dem ersten voreingestellten Zeitraum erfasst wurden.

4. Datenerfassungsverfahren nach einem der Ansprüche 1 bis 3, wobei das Erhalten der erfassten Zieldaten des Zielbenutzers auf der Grundlage des einen oder der mehreren gültigen Zieldatensegmente Folgendes umfasst:
Zusammenfügen aller gültigen Zieldatensegmente basierend auf Erfassungszeitpunkten aller gültigen Zieldatensegmente, um die erfassten Zieldaten zu erhalten.

5. Endgerätevorrichtung (100), umfassend einen Speicher (120), einen Prozessor (180) und ein Computerprogramm, das in dem Speicher gespeichert ist und auf dem Prozessor ausgeführt werden kann, wobei der Prozessor beim Ausführen des Computerprogramms das Datenerfassungsverfahren nach einem der Ansprüche 1 bis 4 implementiert.

6. Computerlesbares Speichermedium, wobei das computerlesbare Speichermedium ein Computerprogramm speichert, und wenn das Computerprogramm von einem Prozessor ausgeführt wird, das Datenerfassungsverfahren nach einem der Ansprüche 1 bis 4 implementiert wird.

## Revendications

1. Procédé de collecte de données mis en œuvre par ordinateur, comprenant :
l'obtention (S31) d'au moins un élément de données collectées d'origine d'un utilisateur cible collectées par un capteur dans une première période de temps prédéfinie, dans lequel les données collectées d'origine comprennent un segment de données valide ;
la sélection (S32), à partir de l'au moins un élément de données collectées d'origine, d'un ou de plusieurs segments de données valides cibles dont la durée totale répond à une exigence de durée prédéfinie ; et
l'obtention (S33) de données collectées cibles de l'utilisateur cible sur la base des un ou plusieurs segments de données valides cibles ;
dans lequel, avant la sélection, à partir de l'au moins un élément de données collectées d'origine, d'un ou de plusieurs segments de données valides cibles dont la durée totale répond à une exigence de durée prédéfinie, le procédé comprend en outre :
la détermination d'une qualité de données des données collectées d'origine ; et
la détermination d'un segment de données dont la qualité de données répond à une exigence de qualité prédéfinie dans les données collectées d'origine en tant que segment de données valide ;
**caractérisé en ce que**
les données collectées d'origine comprennent des données physiologiques humaines prédéfinies et des données de mouvement qui concernent une partie collectée d'un corps humain et qui sont collectées conjointement avec les données physiologiques humaines ; et la détermination d'une qualité de données des données collectées d'origine comprend :
la détermination d'un rapport signal sur bruit des données physiologiques humaines et d'une amplitude de mouvement des données de mouvement ; et
la détermination d'un segment de données dont la qualité de données répond à une exigence de qualité prédéfinie dans les données collectées d'origine en tant que segment de données valide comprend :
la détermination, sur la base de l'amplitude de mouvement des données de mouvement, d'un segment de données cible qui répond à un état de mouvement prédéfini dans les données physiologiques humaines collectées conjointement avec les données de mouvement, dans lequel le segment de données cible répond à l'état de mouvement prédéfini lorsque l'amplitude de mouvement de chaque élément de données de mouvement dans le segment de données cible est inférieure ou égale à un seuil d'amplitude prédéfini ; et la détermination d'un segment de données dont le rapport signal sur bruit est supérieur ou égal à un seuil de rapport signal sur bruit prédéfini dans le segment de données cible en tant que segment de données valide.

2. Procédé de collecte de données selon la revendication 1, dans lequel après l'obtention d'au moins un élément de données collectées d'origine d'un utilisateur cible collectées par un capteur dans une première période de temps prédéfinie, et avant la sélection, à partir de l'au moins un élément de données collectées d'origine, d'un ou de plusieurs segments de données valides cibles dont la durée totale répond à une exigence de durée prédéfinie, le procédé comprend en outre :
si la durée totale des un ou plusieurs segments de données valides dans toutes les données collectées d'origine ne répond pas à l'exigence de durée prédéfinie, la poursuite de l'obtention de nouvelles données collectées d'origine de l'utilisateur cible collectées par le capteur ; et
si un intervalle de temps cible entre un instant de collecte des nouvelles données collectées d'origine et un instant de collecte de données collectées d'origine dont l'instant de collecte est le plus ancien dans l'au moins un élément de données collectées d'origine est supérieur à la durée cible correspondant à la première période de temps prédéfinie, la détermination d'une seconde période de temps prédéfinie sur la base de l'instant de collecte des nouvelles données collectées d'origine et de la durée cible, dans lequel la durée correspondant à la seconde période de temps prédéfinie est égale à la durée cible ; et
la sélection, à partir de l'au moins un élément de données collectées d'origine, d'un ou de plusieurs segments de données valides cibles dont la durée totale répond à une exigence de durée prédéfinie comprend :
la sélection, à partir des données collectées d'origine dont l'instant de collecte se situe dans la seconde période de temps prédéfinie, des un ou plusieurs segments de données valides cibles dont la durée totale répond à l'exigence de durée prédéfinie.

3. Procédé de collecte de données selon la revendication 2, dans lequel après la poursuite de l'obtention de nouvelles données collectées d'origine de l'utilisateur cible collectées par le capteur, si la durée totale du segment de données valide dans toutes les données collectées d'origine ne répond pas à l'exigence de durée prédéfinie, et avant la sélection, à partir des données collectées d'origine dont l'instant de collecte se situe dans la seconde période de temps prédéfinie, des un ou plusieurs segments de données valides cibles dont la durée totale répond à l'exigence de durée prédéfinie, le procédé comprend en outre :
si l'intervalle de temps cible entre l'instant de collecte des nouvelles données collectées d'origine et l'instant de collecte des données collectées d'origine dont l'instant de collecte est le plus ancien dans l'au moins un élément de données collectées d'origine est inférieur ou égal à la durée cible correspondant à la première période de temps prédéfinie, la détermination des nouvelles données collectées d'origine en tant que données collectées d'origine collectées par le capteur dans la première période de temps prédéfinie.

4. Procédé de collecte de données selon l'une quelconque des revendications 1 à 3, dans lequel l'obtention de données cibles collectées de l'utilisateur cible sur la base des un ou plusieurs segments de données valides cibles comprend :
l'épissage de tous les segments de données valides cibles sur la base d'instants de collecte de tous les segments de données valides cibles, pour obtenir les données collectées cibles.

5. Dispositif terminal (100), comprenant une mémoire (120), un processeur (180) et un programme informatique qui est stocké dans la mémoire et qui peut s'exécuter sur le processeur, dans lequel lors de l'exécution du programme informatique, le processeur met en œuvre le procédé de collecte de données selon l'une quelconque des revendications 1 à 4.

6. Support de stockage lisible par ordinateur, dans lequel le support de stockage lisible par ordinateur stocke un programme informatique, et lorsque le programme informatique est exécuté par un processeur, le procédé de collecte de données selon l'une quelconque des revendications 1 à 4 peut être mis en oeuvre.
